(19) Europäisches Patentamt
European Patent Office
Office européen des brevets

(11) Veröffentlichungsnummer: **0 339 518 A2**

(12) **EUROPÄISCHE PATENTANMELDUNG**

(21) Anmeldenummer: **89107281.1**

(22) Anmeldetag: **22.04.89**

(51) Int. Cl.⁴: **C07D 215/12 , C07D 215/20 , B41M 5/12**

(30) Priorität: **28.04.88 DE 3814290**

(43) Veröffentlichungstag der Anmeldung: **02.11.89 Patentblatt 89/44**

(84) Benannte Vertragsstaaten: **CH DE FR GB IT LI**

(71) Anmelder: **BASF Aktiengesellschaft**
**Carl-Bosch-Strasse 38**
**D-6700 Ludwigshafen(DE)**

(72) Erfinder: **Mayer, Udo, Dr.**
**Max-Slevogt-Strasse 27**
**D-6710 Frankenthal(DE)**
Erfinder: **Raulfs, Friedrich-Wilhelm, Dr.**
**Torwiesenstrasse 1**
**D-6800 Mannheim 1(DE)**

(54) **Chinolinverbindungen und deren Verwendung.**

(57) Chinolinverbindungen der allgemeinen Formel I

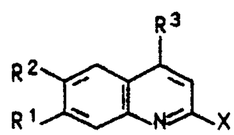

(I),

in der
$R^1$ für Wasserstoff, Alkyl oder Alkoxy, $R^2$ für Alkyl, Cycloalkyl oder Phenylalkyl, $R^3$ für Wasserstoff, Alkyl, gegebenenfalls substituiertes Phenyl und X für einen Rest der Formel

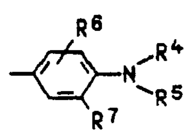

stehen, worin $R^4$ und $R^5$ Wasserstoff, substituiertes Alkyl, Cycloalkyl, Phenyl, Phenylalkyl oder

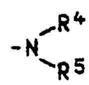

einen 5- oder 6-gliedrigen gesättigten heterocyclischen Ring
$R^6$ Wasserstoff, Alkyl, Alkoxy oder Halogen und $R^7$ Wasserstoff, Chlor, Alkyl oder $R^5$ und $R^7$ ein Brückenglied bedeuten.

Die Verbindungen (I) liefern bereits mit phenolischen Kondensationsprodukten als Elektronenacceptoren gelbe bis orange Färbungen, die farbstärker und in einigen Fällen lichtechter sind als die mit Verbindungen des

EP 0 339 518 A2

nächstliegenden Standes der Technik erhaltenen Färbungen.

## Chinolinverbindungen und deren Verwendung

Aus der DE-A 2 227 597 sind Pyridinverbindungen bekannt, die auf mit Elektronenacceptoren beschichtetem Papier gelbe bis orange Färbungen geben. Aus den EP-A-109 930 und EP-A-159 295 sind Bischinazolinverbindungen bekannt, die als Farbbildner für druck- oder wärmeempfindliche Aufzeichnungsmaterialien geeignet sind. Weiterhin sind aus der JP-A 189 988/1986 Farbbildner auf Basis Dihydroxystyrylchinolin bekannt.

Die Farbbildner des Standes der Technik weisen jedoch in einer Reihe von Anwendungen Nachteile, wie eine geringe Lichtechtheit und/oder geringe Löslichkeit in den zur Verkapselung verwendeten Flüssigkeiten auf. Außerdem entwickeln sie auf Nehmerpapieren mit schwach sauren Acceptoren keine oder nur eine schwache Färbung.

Aufgabe der vorliegenden Anmeldung war es, hinsichtlich der Lichtechtheit der auf Acceptoren gebildeten Färbung, der Löslichkeit und der Nucleophilie verbesserte Farbbildner bereitzustellen.

Die Erfindung betrifft Chinolinverbindungen der allgemeinen Formel I

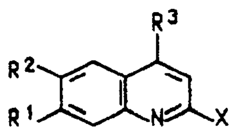

(I),

in der

$R^1$ für Wasserstoff, lineares oder verzweigtes $C_1$-$C_5$-Alkyl oder $C_1$-$C_5$-Alkoxy,

$R^2$ für lineares oder verzweigtes $C_1$-$C_{10}$-Alkyl oder für $C_5$-$C_{10}$-Cycloalkyl, für Wasserstoff, lineares oder verzweigtes $C_1$-$C_{10}$-Alkyl,

$R^3$ $C_5$-$C_{10}$-Cycloalkyl für gegebenenfalls durch Chlor, Brom, Fluor, $C_1$-$C_5$-Alkoxy, $C_1$-$C_5$-Alkyl und/oder

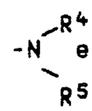 ein-

bis dreifach substituiertes Phenyl oder Phenylakyl mit 7 bis 10 C-Atomen und

X für einen Rest der Formel

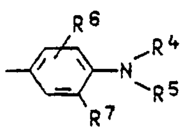

stehen, worin

$R^4$ und $R^5$ unabhängig voneinander Wasserstoff, lineares oder verzweigtes $C_1$-$C_8$-Alkyl, Cyan-$C_2$-$C_4$-alkyl, Halogen-$C_2$-$C_4$-alkyl, $C_5$-$C_{10}$-Cycloalkyl, gegebenenfalls durch Chlor, Brom, Fluor, $C_1$-$C_5$-Alkoxy, Phenyl und/oder $C_1$-$C_5$-Alkyl ein- bis dreifach substituiertes Phenyl oder Phenyl-$C_1$-$C_3$-alkyl oder

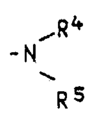 einen

gesättigten 5- oder 6-gliedrigen heterocyclischen Ring,

$R^6$ Wasserstoff, $C_1$-$C_5$-Alkyl, $C_1$-$C_5$-Alkoxy, Chlor, Brom oder Fluor und

$R^7$ Wasserstoff, Chlor oder $C_1$-$C_5$-Alkyl oder $R^7$ zusammen mit $R^5$ ein $R^7$ lineares oder verzweigtes Brückenglied mit insgesamt 2 bis 8 Kohlenstoffatomen bedeuten.

Die Chinolinverbindungen sind schwach gelbe bis farblose Verbindungen, die in Eisessig gelbe bis orange Färbungen liefern. In neutralen oder basischen organischen Lösungsmitteln werden farblose bis

schwach gelbe Lösungen erhalten. Die Farbbildung tritt auch mit Kaolin, Zeolithen, Bentonit, Kieselsäure, Alaun, Zinksalz, Oxalsäure, Phenolen und phenolischen Kondensationsprodukten ein. Aufgrund dieser Eigenschaft eignen sich die Verbindungen (I) als Farbbildner für druck- und wärmeempfindliche Aufzeichnungsmaterialien, insbesondere für die Anwendung in Kopiersystemen.

Die Verbindungen (I) der Erfindung haben den Vorteil, auch auf Papier, das mit phenolischen Kondensationsprodukten beschichtet ist, bereits bei Raumtemperatur farbstarke Färbungen zu liefern. Weiterhin entwickelten einige Chinolinverbindungen (I) Färbungen, die lichtechter sind als Färbungen, die mit den Verbindungen des oben angegebenen Stands der Technik erhalten werden.

Für $R^1$ sind neben Wasserstoff als Alkyl z.B. zu nennen: Methyl, Ethyl, Isopropyl, n-Propyl, n-Butyl, Isobutyl, tert-Butyl und als Alkoxy Methoxy, Ethoxy und Propoxy. Vorzugsweise steht für $R^1$ Wasserstoff, Methyl oder Methoxy.

Als Alkyl mit 1 bis 10 C-Atomen kommen für $R^2$ z.B. Methyl, Ethyl, n-Propyl, Isopropyl, n-Butyl, Isobutyl, tert-Butyl, Hexyl, 2-Ethylhexyl und Octyl und als Cycloalkyl z.B. Cyclohexyl in Betracht. Methyl, Ethyl, Isobutyl und tert-Butyl sind für $R^2$ bervorzugt.

Für $R^3$ sind neben Wasserstoff als $C_1$-$C_{10}$-Alkyl und $C_5$-$C_{10}$-Cycloalkyl z.B. Methyl, Ethyl, Propyl, Isopropyl, Butyl, Isobutyl, Hexyl, Cyclohexyl, 2-Ethylhexyl, Octyl und Decyl, als gegebenenfalls substituiertes Phenyl z.B. Phenyl, 2-, 3- und 4-Chlorphenyl, 3- und 4-Tolyl, 3,5-Xylyl, 3- und 4-Methoxyphenyl und 4-Ethoxyphenyl und als Phenylalkyl mit 7 bis 10 C-Atomen Benzyl, Phenylethyl, 3- oder 4-Methylbenzyl zu nennen. Vorzugsweise ist $R^3$ Methyl, Ethyl, Isopropyl oder Benzyl.

Für $R^4$ und $R^5$ kommen als $C_1$-$C_8$-Alkyl z.B. Methyl, Ethyl, n-Propyl, Isopropyl, n-Butyl, Isobutyl, tert-Butyl, Hexyl, Cyclohexyl, 2-Ethylhexyl und Octyl, als Cyan-$C_2$-$C_4$-alkyl z.B. 2-Cyanethyl, als Halogen-$C_2$-$C_4$-alkyl zum Beispiel 2-Chlorethyl, 2-Bromethyl, 2-Chlorpropyl, 3-Brompropyl und 4-Chlorbutyl und als gegebenenfalls substituiertes Phenyl z.B. Phenyl, 2-Tolyl oder 2-Chlorphenyl oder für Phenylalkyl zum Beispiel Benzyl in Betracht. $R^4$ und $R^5$ stehen vorzugsweise für Methyl, Ethyl, Cyanethyl oder Benzyl.

Weiterhin kann

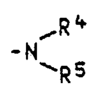

für einen 5- oder 6-gliedrigen, gesättigte heterocyclischen Rest stehen, wie Piperidinyl, Morpholinyl, Piperazinyl, N′-Methylpiperazinyl oder Pyrrolidinyl.

Für $R^6$ sind neben Wasserstoff und den Halogenen Chlor, Brom und Fluor als $C_1$-$C_5$-Alkyl beispielsweise Methyl und Ethyl und als $C_1$-$C_5$-Alkoxy Methoxy zu nennen.

Als Alkyl mit 1 bis 5 C-Atomen kommen für $R^7$ insbesondere Methyl und Ethyl in Betracht. Bevorzugt ist für $R^7$ Wasserstoff. Ist $R^7$ zusammen mit $R^5$ ein lineares oder verzweigtes Brückenglied mit 2 bis 8 Kohlenstoffatomen, dann ist X vorzugsweise ein Rest der Formel

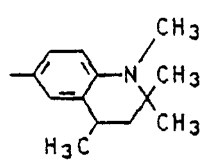

Besonders bevorzugt sind Verbindungen der Formel (I), in denen X für einen der Reste

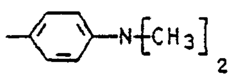 , 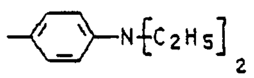 oder 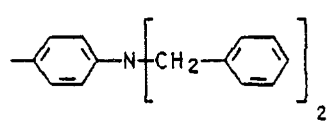

steht.

Wegen ihrer besonders guten Eigenschaften sind die Verbindungen (I), in denen $R^1$ und $R^2$ Methyl sind und $R^3$ und X die in der Tabelle 1 angegebene Bedeutung haben, besonders hervorzuheben.

Tabelle 1

| $R^1$ | X |
|---|---|
| $-CH_3$ | 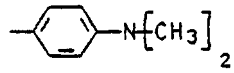 |
| $-C_2H_5$ | 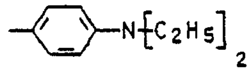 |
| $-CH_2-[CH_3]_2$ | 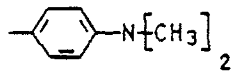 |

Die Chinolinverbindungen (I) erhält man zum Beispiel nach der Friedländer-Synthese durch Umsetzen von 2-Aminophenonen (III)

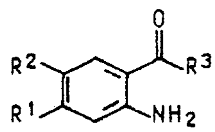

(III)

mit 4-Aminoacetophenonen (IV)

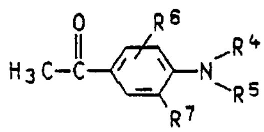

(IV)

in sauren, wasserentziehenden Medien wie Essigsäure, Essigsäureanhydrid, Phosphorsäure, Polyphosphoräure, Schwefelsäure, Zinkchlorid, Thionylchlorid oder Phosphoroxychlorid bei Temperaturen zwischen 50 und 150 °C.

Die Verbindungen (III) können durch Umsetzen eines Säurechlorids (VI) mit dem acylierten Amin (V) in Gegenwart von Aluminiumchlorid oder einer anderen Lewis-Säure, gegebenenfalls in Gegenwart eines Lösungsmittels und unter anschließender Verseifung mit Salzsäure hergestellt werden.

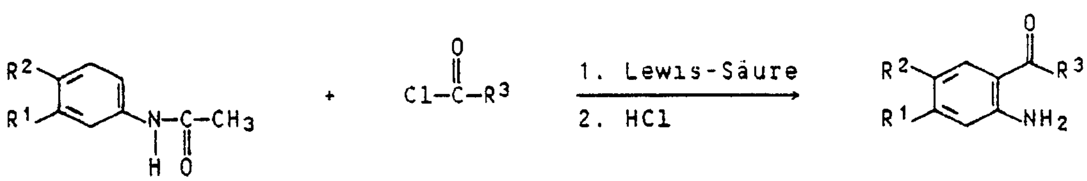


(V) (VI) (III)

Einzelheiten der Herstellung können den Beispielen entnommen werden, in denen sich Angaben über Teile und Prozente, sofern nicht anders vermerkt, auf das Gewicht beziehen.

Beispiel 1

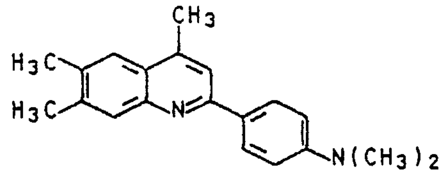

5 g 2-Acetyl-4,5-dimethylanilin und 5 g 4-N,N-Dimethylaminoacetophenon wurden mit 8,2 g Zinkchlorid 3 Stunden bei 110 °C gerührt. Die abgekühlte Reaktionsmasse wurde mit 100 g 2 %iger Salzsäure versetzt und das sich beim Rühren abscheidende, gelbbraune kristalline Zinksalz abfiltriert. Das Zinksalz wurde mit 10 %iger Salzsäure aufgenommen und die Lösung in Gegenwart von Toluol mit 25 %iger Ammoniaklösung auf pH 11 gestellt. Die organische Phase wurde abgetrennt, über Natriumsulfat getrocknet und im Vakuum bis zur Trockene eingeengt. Der Rückstand wurde aus Ethanol umkristallisiert. Ausbeute: 2,2 g 2-(4-N,N-Dimethylaminophenyl)-4,6,7-trimethylchinolin in Form gelblicher Kristalle mit einem Schmelzbereich von 168 bis 171 °C. Eine Lösung dieser Substanz in Toluol ergab auf mit Clay beschicheteten Papieren farbstarke orange Färbungen. Die essigsaure Lösung hat ein Absorptionsmaximum bei $\lambda_{max}$ = 443 nm.

Beispiele 2 bis 4

Es wurde wie in Beispiel 1 verfahren, jedoch wurden anstelle von 4-N,N-Dimethylaminoacetophenon die in Tabelle 2 angegebenen Acetophenone verwendet. Die erhaltenen Farbbildner geben auf Clay die in Spalte 3 angegebene Färbung.

Tabelle 2

| Bsp.-Nr. | Acetophenon | Farbe auf Clay-Papier | $\lambda_{max}$ x) [nm] |
|---|---|---|---|
| 2 | 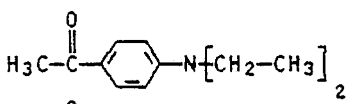 | orange | 454 |
| 3 | 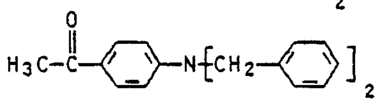 | gelb | 437 |
| 4 | 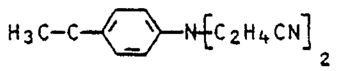 | gelb | 430 |

x) in Essigsäure

Beispiel 5

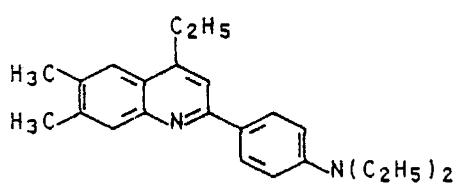

177 g 2-Propionyl-4,5-dimethylanilin und 191 g 4-N,N-Diethylaminoacetophenon wurden in 800 g Eisessig bei 120 bis 125 °C 5 Stunden auf Rückflußtemperatur erhitzt. Das Reaktionsgemisch wurde in 3 l Eiswasser eingerührt und mit 25 %iger Ammoniaklösung auf pH 9 eingestellt. Die überstehende wäßrige Phase wurde abdekantiert und der Rückstand zweimal mit je 100 ml n-Hexan ausgekocht. Nach dem Erkalten wurden die vereinigten Hexanollösungen filtriert und im Vakuum zur Trockene eingedampft, der verbleibende Rückstand wurde destilliert. Bei 0,5 mbar und 230 °C gingen 145 g gelbliches 4-N,N-Diethylaminophenyl-6,7-dimethyl-4-ethylchinolin über. Eine Lösung des Produktes in Trimethylolacrylat ergab sowohl auf mit Clay- als auch mit Phenolharz beschichtetem Papier orange Färbungen. Die essigsaure Lösung dieser Substanz besitzt ein $\lambda_{max}$ von 454 nm.

Beispiel 6 bis 8

Es wurde analog Beispiel 4 verfahren, jedoch wurden anstelle 4-N,N-Diethylaminoacetophenon die in Tabelle 3, Spalte 2 angegebenen Acetophenone verwendet. Die erhaltenen Produkte wurden aus Ethanol umkristallisiert.

Tabelle 3

| Bsp.-Nr. | Acetophenon | Farbe auf Clay-Papier | Schmp. [°C] | $\lambda_{max}$ x) [nm |
|---|---|---|---|---|
| 6 | 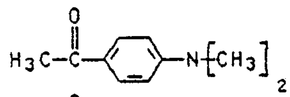 | orange | | 446 |
| 7 | 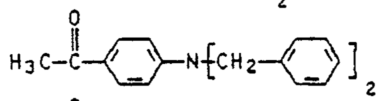 | gelb | | 438 |
| 8 | 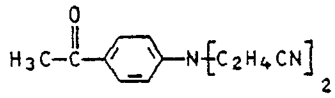 | gelb | | 430 |

x) in Essigsäure

Beispiel 9

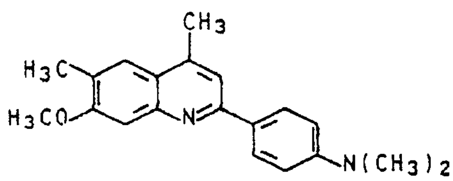

6 g 6-Acetyl-3-methoxy-4-methylanilin und 5,5 g 4-N,N-Dimethylaminoacetophenon wurden mit 9,5 g Zinkchlorid 2,5 Stunden auf 115 °C und 1 Stunde auf 140 °C erhitzt. Die abgekühlte Reaktionsmasse wurde mit 100 ml 2 %iger Salzsäure versetzt, kurz aufgekocht, nach dem Abkühlen abfiltriert und mit Wasser gewaschen. Das braune Zinksalz wurde in 150 ml Toluol und 150 ml 25 %iger Ammoniaklösung eine halbe Stunde unter Rückfluß gekocht. Nach dem Erkalten wurde die organische Phase abgetrennt, über Natriumsulfat getrocknet und das Toluol im Vakuum abdestilliert. Der Rückstand wurde über Kieselgel 60 (Korngröße unter 0,063 mm der Fa. Merck) mit Toluol-Essigsäureethylester im Volumenverhältnis 3:1 chromatographiert. Nach Umkristallisieren aus Ethanol wurden 2,44 g 2-(4-N,N-Dimethylaminophenyl)-4,6-dimethyl-7-methoxy-chinolin erhalten. Die essigsaure Lösung hat ein $\lambda_{max}$ bei 442 nm. Eine Lösung des Produktes in Toluol ergab sowohl auf mit Clay- als auch mit Phenolharz beschichtetem Papier gelbe Färbungen.
Schmp. 197 bis 198,5 °C.

Beispiel 10

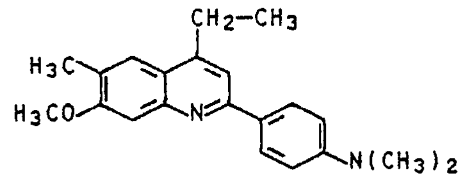

Es wurde analog Beispiel 7 verfahren, jedoch wurde anstelle von 6-Acetyl-3-methoxy-4-methylanilin die äquivalente Menge 6-Propionyl-3-methoxy-4-methylanilin verwendet. Nach dem Aufarbeiten wie in Beispiel 7 wurde 2-(4-N,N-Dimethylaminophenyl)-4-ethyl-7-methoxy-6-methylchinolin erhalten. Dieses Chinolinderivat hat ein $\lambda_{max}$ bei 443 nm.

Beispiel 11

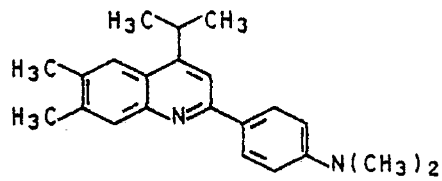

220 g 2-Isobutyryl-4,5-dimethylanilin und 137 g 4-N,N-Dimethylaminoacetophenon, wurden in 800 g Eisessig eine Stunde auf 120 °C, dann 4 Stunden auf 130 °C und weitere 2 Stunden auf 135 °C unter Rühren erhitzt. Das Reaktionsgemisch wurde auf 3 l Eiswasser gegeben und mit konzentrierter Ammoniaklösung alkalisch gestellt. Die überstehende wäßrige Phase wurde abdekantiert und der schmierige Rückstand mit 3 l 1 %iger Salzsäurelösung behandelt. Der Niederschlag wurde abfiltriert, mit Wasser gewaschen und mit 2 %iger Ammoniaklösung aufgekocht. Die überstehende wäßrige Phase wurde abdekantiert und der braune Rückstand mit 1000 ml siedendem n-Hexan extrahiert. Die heiße n-Hexanlösung wurde filtriert. In der Kälte schieden sich 81 g 2-(4-N,N-Dimethylaminophenyl)-6,7-dimethyl-4-propylchinolin ab. Eine Lösung dieses Produktes in Trimethylolpropantriacrylat ergab sowohl auf Clay- als auch auf mit Phenolharz beschichtetem Papier orange Färbungen. Die essigsaure Lösung dieser Substanz hat ein $\lambda_{max}$ bei 446 nm.

Beispiel 12

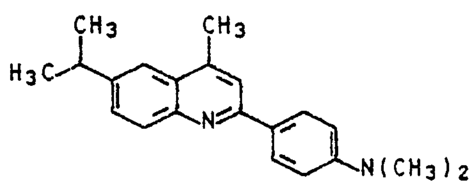

16,5 g 2-Acetyl-4-isopropylanilin und 8,15 g 4-N,N-Dimethylaminoacetophenon wurden mit 14,4 g Zinkchlorid 2,5 Stunden auf 115 °C und eine weitere Stunde auf 140 °C erhitzt. Nach dem Aufarbeiten wie in Beispiel 7 wurde 2-(4-N,N-Dimethylaminophenyl)-6-isopropyl-4-methylchinolin erhalten. Dieses Chinolinderivat hat in Eisessig ein $\lambda_{max}$ bei 448 nm.

Beispiel 13

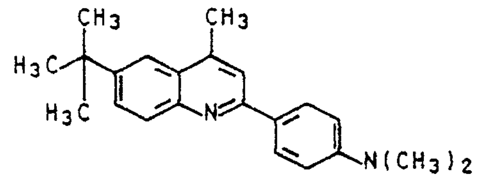

Es wurde analog Beispiel 10 verfahren, jedoch wurde anstelle von 2-Acetyl-4-isopropylanilin die äquivalente Menge 2-Acetyl-4-tert-butylanilin verwendet. Nach dem Aufarbeiten wie in Beispiel 7 wurde 2-(4-N,N-Dimethylaminophenyl)-6-tert-butyl-4-methylanilin erhalten. Dieses Chinolinderivat hat in Eisessig ein $\lambda_{max}$ bei 447 nm.

Beispiel 14

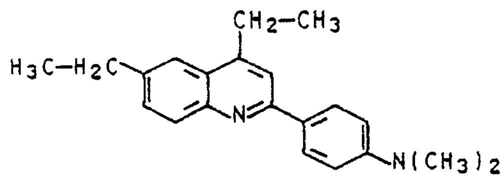

27,05 g 4-Ethyl-2-propionylanilin und 16,3 g 4-N,N-Dimethylaminoacetophenon wurden mit 28 g Zinkchlorid 3 Stunden auf 118 °C erhitzt. Nach dem Aufarbeiten analog Beispiel 7 wurde 2-(4-N,N-Dimethylaminophenyl)-4,6-diethylchinolin erhalten. Die essigsaure Lösung hat ein $\lambda_{max}$ bei 448 nm. Eine Lösung des Produktes in Toluol ergab sowohl mit Clay- als auch mit Phenolharz beschichtetem Papier orange Färbungen.

**Ansprüche**

1. Chinolinverbindungen der allgemeinen Formel I

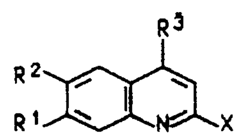

(I),

in der

$R^1$ für Wasserstoff, lineares oder verzweigtes $C_1$-$C_5$-Alkyl oder $C_1$-$C_5$-Alkoxy,

$R^2$ für lineares oder verzweigtes $C_1$-$C_{10}$-Alkyl oder für $C_5$-$C_{10}$-Cycloalkyl,

$R^3$ für Wasserstoff, lineares oder verzweigtes $C_1$-$C_{10}$-Alkyl, $C_5$-$C_{10}$-Cycloalkyl, für gegebenenfalls durch Chlor, Brom, Fluor, $C_1$-$C_5$-Alkoxy, $C_1$-$C_5$-Alkyl und/oder

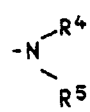

ein- bis dreifach substituiertes Phenyl oder Phenylalkyl mit 7 bis 10 C-Atomen und

X für einen Rest der Formel

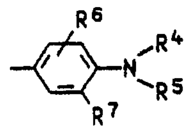

stehen, worin

$R^4$ und $R^5$ unabhängig voneinander Wasserstoff, lineares oder verzweigtes $C_1$-$C_8$-Alkyl, Cyan-$C_2$-$C_4$-alkyl, Halogen-$C_2$-$C_4$-alkyl, $C_5$-$C_{10}$-Cycloalkyl, gegebenenfalls durch Chlor, Brom, Fluor, $C_1$-$C_5$-Alkoxy, Phenyl und/oder $C_1$-$C_5$-Alkyl ein- bis dreifach substituiertes Phenyl oder Phenyl-$C_1$-$C_3$-alkyl oder

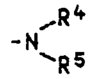

einen gesättigten 5- oder 6-gliedrigen heterocyclischen Ring,

$R^6$ Wasserstoff, $C_1$-$C_5$-Alkyl, $C_1$-$C_5$-Alkoxy, Chlor, Brom oder Fluor und

$R^7$ Wasserstoff, Chlor oder $C_1$-$C_5$-Alkyl oder $R^7$ zusammen mit $R^5$ ein lineares oder verzweigtes Brückenglied mit insgesamt 2 bis 8 Kohlenstoffatomen bedeuten.

2. Chinolinverbindungen gemäß Anspruch 1, dadurch gekennzeichnet, daß $R^1$ und $R^2$ jeweils für Methyl stehen.

3. Chinolinverbindungen gemäß Anspruch 1 oder 2, dadurch gekennzeichnet, daß X für

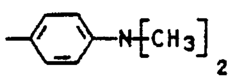 , 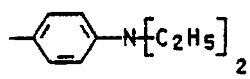 oder 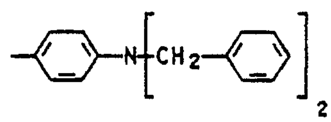

steht.

4. Verwendung der Chinolinverbindungen gemäß den Ansprüchen 1, 2 oder 3 als Farbbildner in druckempfindlichen Aufzeichnungssystemen.